(19) Europäisches Patentamt
European Patent Office
Office européen des brevets

(11) Veröffentlichungsnummer: **0 180 838**
**B1**

(12) **EUROPÄISCHE PATENTSCHRIFT**

(45) Veröffentlichungstag der Patentschrift: **16.05.90**

(21) Anmeldenummer: **85113312.4**

(22) Anmeldetag: **21.10.85**

(51) Int. Cl.$^5$: **C 07 D 249/08,**
C 07 D 233/60,
C 07 D 403/08,
C 07 D 405/14,
C 07 D 409/14,
C 07 D 405/08,
C 07 D 409/08,
A 01 N 43/653, A 01 N 43/50,
A 01 N 43/08, A 01 N 43/10

(54) **Substituierte Azolylcyclopropyl-azolylmethyl-carbinol-Derivate.**

(30) Priorität: **02.11.84 DE 3440117**

(43) Veröffentlichungstag der Anmeldung:
**14.05.86 Patentblatt 86/20**

(45) Bekanntmachung des Hinweises auf die Patenterteilung:
**16.05.90 Patentblatt 90/20**

(84) Benannte Vertragsstaaten:
**AT BE CH DE FR GB IT LI NL SE**

(56) Entgegenhaltungen:
**EP-A-0 044 605**
**EP-A-0 120 276**
**EP-A-0 122 056**
**EP-A-0 122 693**
**EP-A-0 164 246**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Regel, Erik, Dipl.-Ing.**
**Untere Bergerheide 26**
**D-5600 Wuppertal 1 (DE)**
Erfinder: **Böckmann, Klaus, Dr.**
**Andreas-Gryphius-Strasse 7**
**D-5000 Köln 80 (DE)**
Erfinder: **Büchel, Karl Heinz, Prof. Dr.**
**Dabringhausener Strasse 42**
**D-5093 Burscheid (DE)**
Erfinder: **Lürssen, Klaus, Dr.**
**August-Kierpsel-Strasse 151**
**D-5060 Bergisch-Gladbach (DE)**
Erfinder: **Brandes, Wilhelm, Dr.**
**Eichendorffstrasse 3**
**D-5653 Leichlingen 1 (DE)**
Erfinder: **Konze, Jörg, Dr.**
**Magazinstrasse 61**
**D-5000 Köln 90 (DE)**
Erfinder: **Reinecke, Paul, Dr.**
**Steinstrasse 8**
**D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 180 838 B1

(56) Entgegenhaltungen:

**JOURNAL OF ORGANIC CHEMISTRY, Band 48, 1983, Seiten 5133-5134 K. OKUMA et al.: "Reaction of dimethyloxosulfonium methylide with epoxides. Preparation of oxetanes."**

## Beschreibung

Die vorliegende Erfindung betrifft neue substituierte Azolylcyclopropyl-azolylmethyl-carbinol-Derivate, mehrere Verfahren zu ihrer Herstellung sowie ihre Verwendung als Pflanzenwachstumsregulatoren und Fungizide.

Es ist bereits bekannt geworden, daß bestimmte Diazolyl-Derivate fungizide und pflanzenwuchsregulierende Eigenschaften besitzen (vgl. EP—A—O 044 605). So lassen sich beispielsweise 1,3-Di-(1,2,4-triazol-1-yl)-2-(2-chlorophenyl)-propan-2-ol, 1,2-Di-(1,2,4-triazol-1-yl)-2-(3-chlorophenyl)-propan-2-ol, 1,3-Di-(1,2,4-triazol-1-yl)-2-(4-chlorophenyl)-propan-2-ol und 1,3-Di-(1,2,4-triazol-1-yl)-2-phenyl-propan-2-ol zur Bekämpfung von Pilzen und zur Regulierung des Pflanzenwachstums verwenden. Die Wirkung dieser Stoffe ist jodoch, vor allem bei niedrigen Aufwandmengen und Aufwandkonzentrationen, nicht immer ganz befriedigend.

Weiterhin sind aus der EP—A—O 120 276 und der EP-A 0 122 693 Bis-azolyl-carbinole mit antimykotischen bzw. fungiziden Eigenschaften bekannt. In diesen Druckschriften werden jedoch keine entsprechenden Stoffe offenbart, die eine Azolyl-cyclo-propyl-Gruppe enthalten.

Schließlich wurden in der EP—A—O 164 246 schon fungizid wirksame Azolylcyclopropyl-azolylmethyl-Carbinol-Derivate beschrieben. Es werden aber keine Verbindungen erwähnt, in denen zwei 1,2,4-Triazolyl-Reste vorhanden sind und gleichzeitig das zentrale Carbinol-Kohlenstoffatom mit einem Naphthyl-Rest oder mit einem Phenyl-Rest verbunden ist, der seinerseits durch Methylthio, Trifluormethoxy, gegebenenfalls substituiertes Phenyl oder gegebenenfalls substituiertes Phenoxy substituiert ist. Außerdem werden auch keine Verbindungen dieses Typs aufgeführt, in denen einer der Azolyl-Reste für Imidazolyl steht.

Es wurden nun neue substituierte Azolylcyclopropyl-azolylmethyl-carbinol-Derivate der Formel

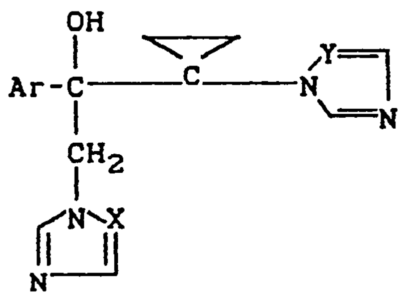

(I)

in welcher

a) X für ein Stickstoffatom oder eine CH—Gruppe steht,
Y für eine CH—Gruppe steht und
Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder
Ar für Naphthyl steht, oder

b) X für eine CH—Gruppe steht,
Y für ein Stickstoffatom steht und
Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder
Ar für Naphthyl steht, oder

c) X für ein Stickstoffatom steht,
Y für ein Stickstoffatom steht und
Ar für Phenyl steht, das einfach oder zweifach, gleichartig oder verschieden substituiert sein kann durch Methylthio, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder
Ar für Naphthyl steht,

sowie deren Säureadditions-Salze und Metallsalz-Komplexe gefunden.

Weiterhin wurde gefunden, daß man substituierte Azolylcyclopropyl-azolylmethyl-carbinol-Derivate der Formel (I) sowie deren Säureadditions-Salze und Metallsalz-Komplexe erhält, wenn man

a) in einer ersten Stufe Aryl-azolylcyclopropyl-ketone der Formel

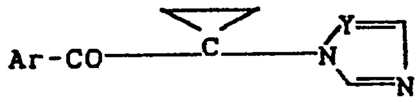

(II)

in welcher

Ar und Y die oben angegebene Bedeutung haben, mit Dimethyloxosulfonium-methylid der Formel

$$\overset{\delta+\ \delta-}{(CH_3)_2SOCH_2} \qquad (III)$$

in Gegenwart eines Verdünnungsmittels umsetzt und die dabei entstehenden Aryl-azolylcyclopropyl-oxirane der Formel

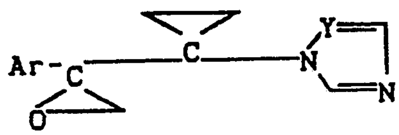

(IV)

in welcher

Ar und Y die oben angegebene Bedeutung haben, in einer zweiten Stufe mit Azolen der Formel

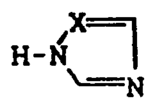

(V)

in welcher

X die oben angegebene Bedeutung hat, in Gegenwart eines Verdünnungsmittels und in Gegenwart einer Base umsetzt; oder

b) Diazolyl-Keto-Derivate der Formel

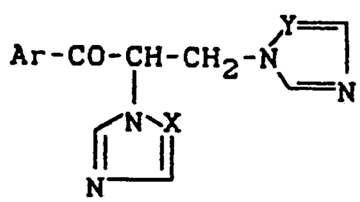

(VI)

in welcher

Ar, X und Y die oben angegebene Bedeutung haben, mit Dimethyloxosulfonium-methylid der Formel

$$\overset{\delta+\ \delta-}{(CH_3)_2SOCH_2} \qquad (III)$$

in Gegenwart eines Verdünnungsmittels umsetzt,
und gegebenenfalls anschließend an die so erhaltenen Verbindungen der Formel (I) eine Säure oder ein Metallsalz addiert.

Schließlich wurde gefunden, daß die neuen substituierten Azolylcyclopropyl-azolylmethyl-carbinol-Derivate der Formel (I) sowie deren Säureadditions-Salze und Metallsalz-Komplexe starke pflanzenwuchsregulierende und fungizide Eigenschaften besitzen.

Überraschenderweise zeigen die erfindungsgemäßen Stoffe eine bessere pflanzenwuchsregulierende und fungizide Wirksamkeit als die konstitutionell ähnlichen, aus dem Stand der Technik vorbekannten Diazolyl-Derivate 1,3-Di-(1,2,4-triazol-1-yl)-2-(2-chlor-phenyl)-propan-2-ol, 1,3-Di-(1,2,4-triazol-1-yl)-2-(3-chlor-phenyl)-propan-2-ol, 1,3-Di-(1,2,4-triazol-1-yl)-2-(4-chlor-phenyl)-propan-2-ol und 1,3-Di-(1,2,4-triazol-1-yl)-2-phenylpropan-2-ol.

Die erfindungsgmäßen substituierten Azolylcyclopropyl-azolylmethyl-carbinol-Derivate sind durch die Formel (I) allgemein definiert. In dieser Formel stehen Ar, X und Y für die Bedeutungen, die bereits für diese Reste genannt wurden.

Erfindungsgemäße Verbindungen sind auch Additionsprodukte aus Säuren und substituierten Azolyl-Cyclopropyl-azolylmethyl-Carbinol-Derivaten der Formel (I), in welcher Ar, X und Y die oben angegebenen Bedeutungen haben.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bersteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Erfindungsgemäße Verbindungen sind auch Additionsprodukte aus Salzen von Metallen der II. bis IV. Haupt- und der I. und II. sowie IV. bis VIII. Nebengruppe des Periodensystems der Elemente und substituierten Azolylcyclopropyl-azolylmethyl-carbinol-Derivaten der Formel (I), in denen Ar, X und Y die oben angegebenen Bedeutungen haben. Hierbei sind Salze des Kupfers, Zinks, Mangans, Magnesiums, Zinns, Eisens und des Nickels bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in der folgenden Tabelle aufgeführten Verbindungen genannt:

Tabelle 1

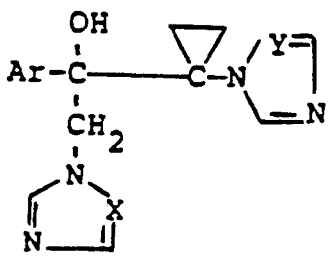

(I)

| Ar | X | Y |
|---|---|---|
| 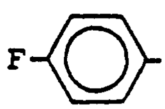 | N | CH |
| 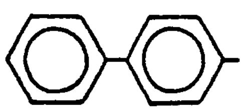 | N | CH |
| 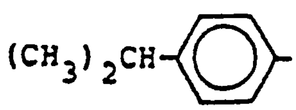 | N | CH |
| 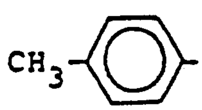 | N | CH |
| 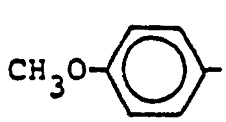 | N | CH |

Tabelle 1 (Fortsetzung)

| Ar | X | Y |
|---|---|---|
| 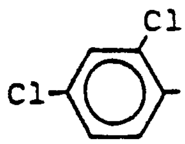 | N | CH |
| 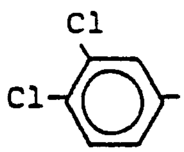 | N | CH |
| 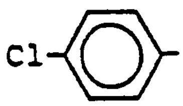 | CH | N |
| 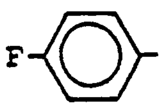 | CH | N |
| 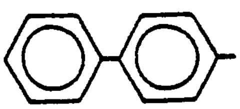 | CH | N |
| 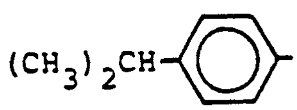 | CH | N |
| 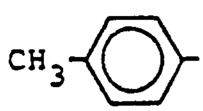 | CH | N |
| 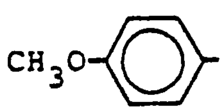 | CH | N |
| 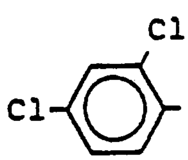 | CH | N |
| 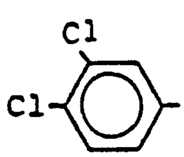 | CH | N |

Tabelle 1 (Fortsetzung)

| Ar | X | Y |
|---|---|---|
| 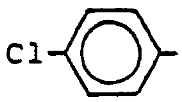 | CH | CH |
| 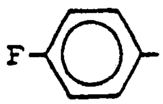 | CH | CH |
| 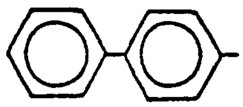 | CH | CH |
| 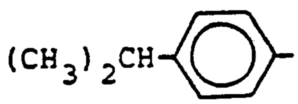 | CH | CH |
| 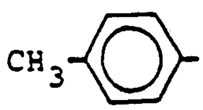 | CH | CH |
| 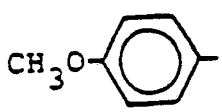 | CH | CH |
| 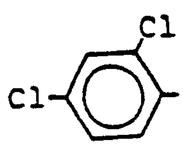 | CH | CH |
| 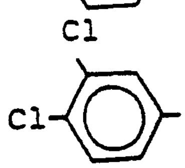 | CH | CH |

Die für das erfindungsgemäße Verfahren (a) als Ausgangsstoffe zu verwendenden Aryl-azolylcyclopropyl-ketone sind durch die Formel (II) allgemein definiert. In dieser Formel haben Ar und Y die Bedeutungen, die bereits im Zusammenhang mit der Beschreibung der erfindungsgemäßen Stoffe der Formel (I) für diese Substituenten genannt wurden.

Die Aryl-azolylcyclopropyl-ketone der Formel (II) sind neu. Sie werden erhalten, indem man Aryl-halogenpropylketone der Formel

$$Ar{-}CO{-}\underset{\displaystyle Hal'}{\underset{|}{CH}}{-}CH_2CH_2{-}Hal'' \qquad (VII)$$

in welcher

Ar die oben angegebene Bedeutung hat und

Hal' und Hal'' für Halogen, vorzugsweise Brom oder Chlor stehen, mit Azolen der Formel

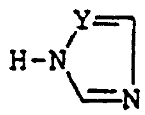

(V)

in welcher

Y die oben angegebene Bedeutung hat,

in Gegenwart eines Verdünnungsmittels und in Gegenwart einer Base umsetzt.

Als Verdünnungsmittel kommen für die Herstellung der Ketone der Formel (II) unter den Reaktionsbedingungen inerte organische Lösungsmittel in Frage, Hierzu gehören vorzugsweise Alkohole, wie z.B. Ethanol, Methoxyethanol oder Propanol; Ketone, wie z.B. Aceton und 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für diese Umsetzung alle üblicherweise verwendbaren anorganischen oder organischen Basen in Betracht. Hierzu gehören vorzugsweise Alkalicarbonate, wie z.B. Natrium- und Kaliumcarbonat; Alkalihydoxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung dieser Umsetzung in einem größeren Bereich variiert werden. Im allgemeinen arbeitet man bei Temperaturen zwischen 0°C und 200°C, vorzugweise zwischen 60°C und 150°C.

Bei der Durchführung dieser Umsetzung setzt man vorzugsweise auf 1 Mol Aryl-halogenpropyl-keton der Formel (VII) 1 bis 2 Mol Azol der Formel (V) und gegebenenfalls 1 bis 2 Mol Base ein. Die Isolierung der Zwischenprodukte der Formel (II) erfolgt in allgemein üblicher Weise.

Die Aryl-halogenpropyl-ketone der Formel (VII) sind bekannt oder lassen sich nach üblichen Methoden in einfacher Weise herstellen (vgl. DE—A—25 21 104, DE—A—23 20 355 und DE—A—23 51 948).

Die neuen Aryl-azolylcyclopropyl-ketone der Formel (II) eignen sich nicht nur als Ausgangsstoffe für das erfindungsgemäße Verfahren (a), sondern stellen allgemein interessante Zwischenprodukte dar.

Das sowohl bei dem erfindungsgemäßen Verfahren (a) als auch bei dem erfindungsgemäßen Verfahren (b) als Reaktionskomponente benötigte Dimethyloxosulfonium-methylid der Formel (III) ist bekannt (vgl. J. Am. Chem. Soc. *87,* 1363—1364 (1965)). Es wird bei den obigen Umsetzungen in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriumamid, in Gegenwart eines Verdünnungsmittels erzeugt.

Die bei dem erfindungsgemäßen Verfahren (a) als Zwischenprodukte auftretenden Aryl-azolylcyclopropyl-oxirane der Formel (IV) sind noch nicht bekannt. Sie stellen allgemein intersessante Zwischenprodukte dar.

Die außerdem für die zweite Stufe des erfindungsgemäßen Verfahrens (a) als Ausgangsstoffe benötigten Azole der Formel (V) sind allgemein bekannte Verbindungen der organischen Chemie.

Die für das erfindungsgemäße Verfahren (b) als Ausgangsstoffe zu verwendenden Di-azolyl-Keto-Derivate sind durch die Formel (VI) allgemein definiert. In dieser Formel haben Ar, X und Y die Bedeutungen, die bereits im Zusammenhang mit der Beschreibung der erfindungsgemäßen Stoffe der Formel (I) für diese Substituenten genannt wurden.

Die Di-azolyl-Keto-Derivate der Formel (VI) sind neu. Sie werden erhalten, indem man Aryl-azolylmethyl-ketone der Formel

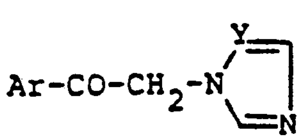

(VIII)

in welcher

Ar und Y die oben angegebene Bedeutung haben,

mit Hydroxymethylazolen der Formel

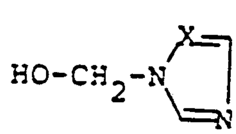

(IX)

in welcher

X die oben angegebene Bedeutung hat,

in Gegenwart eines Katalysators und in Gegenwart eines Verdünnungsmittels umsetzt.

Als Verdünnungsmittel kommen für dieses Verfahren zur Herstellung der Di-azolyl-keto-Derivate der Formel (VI) vorzugsweise inerte organische Lösungsmittel in Frage. Hierzu gehören vorzugsweise Alkohole, wie Methanol und Ethanol; Ether, wie Tetrahydrofuran und Dioxan; aromatische Kohlenwasserstoffe, wie Benzol und Toluol; sowie halogenierte aliphatische und aromatische Kohlenwasserstoffe, wie Methylenchlorid, Chloroform, Chlorbenzol und Dichlorbenzol.

Dieses Verfahren wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen, wie Pyridin und Piperidin; sowie insbesondere Piperidinacetat.

Die Reaktionstemperaturen können bei der Durchführung dieses Verfahrens in einem größeren Bereich variiert werden. Im allgemeinen arbeitet man zwischen 20 und 160°C, vorzugsweise bei der Siedetemperatur des jeweiligen Lösungsmittels.

Bei der Durchführung dieses Verfahrens setzt man auf 1 Mol Keton der Formel (VIII) 1 bis 1,5 Mol Hydroxymethylazol der Formel (IX) und katalytische bis 0,2 molare Mengen an Katalysator ein.

Die Aryl-azolylmethyl-ketone der Formel (VIII) sind bekannt (vgl. z.B. DE—A—24 31 407, DE—A—26 10 022 und DE—A—26 38 470).

Die Hydroxymethylazole der Formel (IX) sind ebenfalls bekannt (vgl. EP—A—0 006 102 sowie Chem. Heterocycl. Comp. *1980,* 189).

Die Di-azolyl-keto-Derivate der Formel (VI) stellen interessante Zwischenprodukte dar und zeigen in entsprechenden Aufwandmengen bzw. -konzentrationen auch fungizide und pflanzenwachstumsregulierende Eigenschaften.

Als Verdünnungsmittel kommen für die erste Stufe des erfindungsgemäßen Verfahrens (a) inerte organische Lösungsmittel in Frage. Hierzu gehören vorzugsweise Ether, wie Tetrahydrofuran oder Dioxan, aliphatische und aromatische Kohlenwasserstoffe, wie insbesondere Benzol, Toluol oder Xylol; sowie Dimethylsulfoxid.

Die Reaktionstemperaturen können bei der Durchführung der ersten Stufe des erfindungsgemäßen Verfahrens (a) in einem größeren Bereich variiert werden. Im allgemeinen arbeitet man zwischen 0 und 100°C, vorzugsweise zwischen 10 und 60°C.

Bei der Durchführung der ersten Stufe des erfindungsgemäßen Verfahrens (a) setzt man auf 1 Mol Aryl-azolylcyclopropyl-keton der Formel (II) vorzugsweise 1 bis 3 Mol Dimethyloxosulfonium-methylid der Formel (III) ein, in situ erzeugt aus Trimethyloxosulfoniumiodid in Dimethylsulfoxid und Natriumhydrid. Die Isolierung der Zwischenprodukte der Formel (IV) erfolgt in allgemein üblicher Art und Weise.

Als Verdünnungsmittel kommen für die zweite Stufe des erfindungsgemäßen Verfahrens (a) inerte organische Lösungsmittel in Frage. Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid; sowie Hexamethylphosphorsäuretriamid.

Die zweite Stufe des erfindungsgemäßen Verfahrens (a) wird in Gegenwart einer Base durchgeführt. Dabei kommen alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung der zweiten Stufe des erfindungsgemäßen Verfahrens (a) in einem größeren Bereich variiert werden. Im allgemeinen arbeitet man bei Temperaturen zwischen 0°C und 200°C, vorzugsweise zwischen 60°C und 150°C.

Bei der Durchführung der zweiten Stufe des erfindungsgemäßen Verfahrens (a) setzt man vorzugsweise auf 1 Mol Oxiran der Formel (IV) 1 bis 2 Mol Azol der Formel (V) und 1 bis 2 Mol Base ein. Die Isolierung der Endprodukte erfolgt in allgemein üblicher Weise.

Die Reaktionsbedingungen bei der Durchführung des erfindungsgemäßen Verfahrens (b) entsprechen denen für die Durchführung der ersten Stufe des Verfahrens (a).

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten

Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchschemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe Pipelines oder überiandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide, Hierdurch wird die Gefahr des Umknickens ("Lagerns") der Pflanzen vor der Ernte verringert oder vollkommen beseitigt.

Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kulter leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in machen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung de Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der

Pflanzen beeinflußt werden, so daß de Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schleißlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plazmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Mehltau, Rost, Septoria und Fusariumarten sowie Pyrenophora teres; ferner zur Bekämpfung von Reiskrankheiten, wie Pyricularia oryzae; sowie von Venturia-Arten, wie Venturia inaequalis, eingesetzt werden. Außerdem ziegen dei erfindungsgemäßen Stoffe ein breites und gutes fungizides in-vitro-Wirkungsspektrum.

In entsprechenden Aufwandmengen zeigen die erfindungsgemäßen Stoffe auch eine herbizide Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Acetone, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen- Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Aso- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Dungemitteln und enderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem gröberen Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeistraum vorgenommen wird, dessen genaue Abgrenzung sich nach den klimatischen und vegetativen Gegebenheiten richtet.

Auch beim Ensatz der erfindungsgemäßen Stoffe als Fungizide kann die Aufwandmenge de nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02%, am Wirkungsort erforderlich.

Die Herstellung der erfindungsgemäßen Wirkstoffe wird durch die folgenden Beispiele veranschaulicht.

## Herstellungsbeispiele

### Beispiel 1

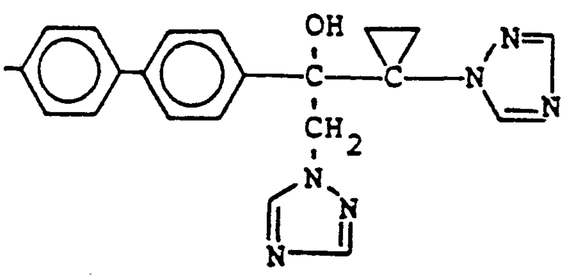

(I-1)

Schmelzpunkt: 184°C

Tabelle 2

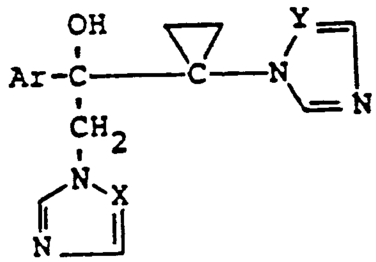

(I)

| Bsp.-Nr. | Ar | R | X | Y | Schmelzpunkt (°C) |
|---|---|---|---|---|---|
| I- 2 | 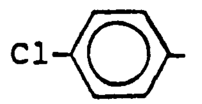 | H | N | CH | 196 |
| I- 3 | 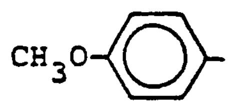 | H | CH | N | 213 |

Beispiel II-1

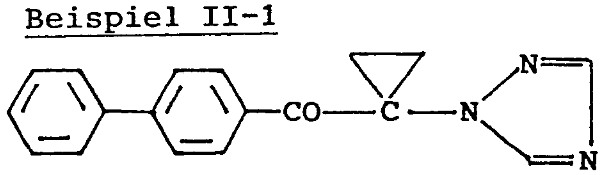

Schmelzpunkt: 169°C

Beispiel IV-1

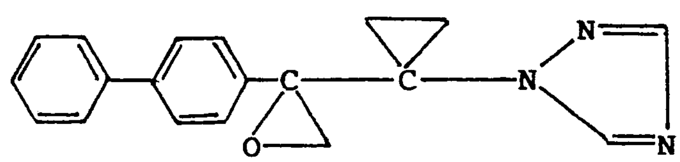

Öl

Beispiel VI-1

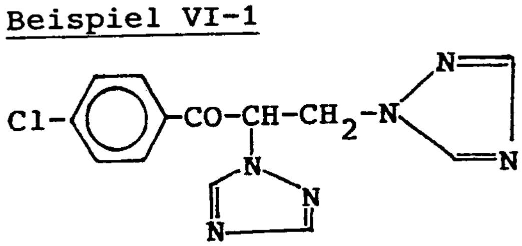

(VI-1)

Ein Gemisch aus 44,3 g 4-Chlorphenyl-(1,2,4-triazol-1-yl-methyl)-keton, 19,6 g 1-Hydroxymethyl-1,2,4-triazol, 300 ml Toluol, 6 g Essigsäure und 2 ml Piperidin wird am Wasserabscheider bis zur beendeten Wasserabscheidung erhitzt. Man läßt erkalten, saugt den entstandenen kristallinen Niederschlag ab und wäscht ihn mit Diisopropylether nach. Man erhält 47 g (76% der Theorie) 1-(4-Chlorphenyl)-2,3-di-(1,2,4-triazol-1-yl)-1-propanon vom Schmelzpunkt 204°C.

Entsprechend Beispiel VI—1 und gemäß den angegebenen Verfahrensbedingungen werden die in der folgenden Tabelle aufgeführten Vorprodukte der Formel (VI) erhalten.

Tabelle 2

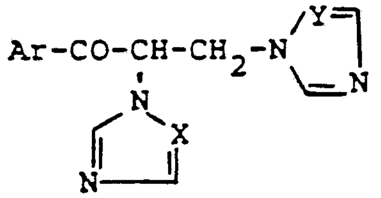

(VI)

| Bsp.-Nr. | Ar | X | Y | Schmelzpunkt (°C) bzw. Brechungsindex |
|---|---|---|---|---|
| VI-2 | 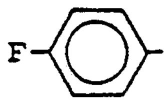 | N | N | 185 |
| VI-3 | 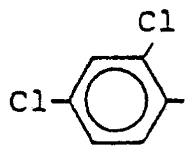 | N | N | $n_D^{20}$ = 1,5504 |
| VI-4 | 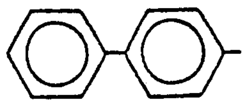 | N | N | 195 |
| VI-5 | 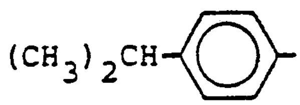 | N | N | 170 |
| VI-6 | 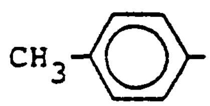 | N | N | 212 |
| VI-7 | 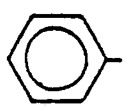 | N | N | 170 |

**Patentansprüche**

1. Substituierte Azolylcyclopropyl-azolylmethyl-carbinol-Derivate der Formel

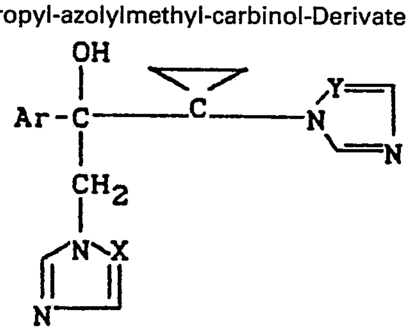

(I)

in welcher

a) X für ein Stickstoffatom oder eine CH-Gruppe steht,

Y für eine CH-Gruppe steht und

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

b) X für eine CH-Gruppe, steht,

Y für ein Stickstoffatom steht und

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

c) X für ein Stickstoffatom steht,

Y für ein Stickstoffatom steht und

Ar für Phenyl steht, das einfach oder zweifach, gleichartig oder verschieden substituiert sein kann durch Methylthio, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, sowie deren Säureadditions-Salze und Metallsalzkomplexe.

2. Verfahren zur Herstellung von substituierten Azolylcyclopropyl-azolylmethyl-carbinol-Derivaten der Formel

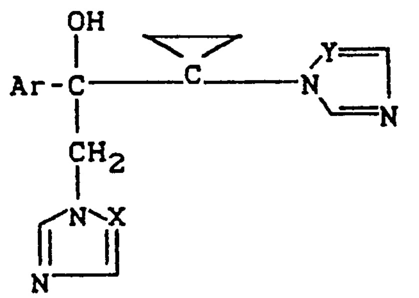

(I)

in welcher

a) X für ein Stickstoffatom oder eine CH-Gruppe steht,

Y für eine CH-Gruppe steht und

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

b) X für eine CH-Gruppe, steht,

Y für ein Stickstoffatom steht und

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

c) X für ein Stickstoffatom steht,

Y für ein Stickstoffatom steht und

Ar für Phenyl steht, das einfach oder zweifach, gleichartig oder verschieden substituiert sein kann durch Methylthio, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, sowie von Säureadditions-Salze und Metallsalzkomplexe von Verbindungen der Formel (1), dadurch gekennzeichnet, daß man.

a) in einer ersten Stufe Aryle-azolylcyclopropylketone der Formel

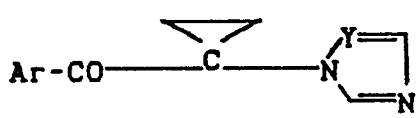

(II)

in welcher
Ar und Y die oben angegenbene Bedeutung haben, mit Dimethyloxosulfonium-methylid der Formel

$$\overset{\delta+\ \delta-}{(CH_3)_2SOCH_2} \qquad (III)$$

in Gegenwart eines Verdünnungsmittels umsetzt und die dabei entstehenden Aryl-azolylcyclopropyl-oxirane der Formel

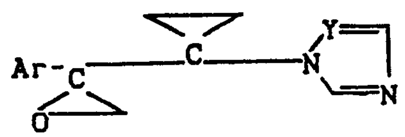

(IV)

in welcher
Ar und Y die oben angegebene Bedeutung haben, in einer zweiten Stufe mit Azolen der Formel

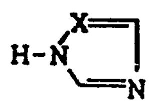

(V)

in welcher
X die oben angegebene Bedeutung hat, in Gegenwart eines Verdünnungsmittels und in Gegenwart einer Base umsetzt; oder
b) Diazolyl-Keto-Derivate der Formel

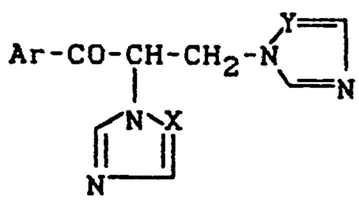

(VI)

in welcher
Ar, X und Y die oben angegebene Bedeutung haben, mit Dimethyloxosulfonium-methylid der Formel

$$\overset{\delta+\ \delta-}{(CH_3)_2SOCH_2} \qquad (III)$$

in Gegenwart eines Verdünnungsmittels umsetzt, und gegebenenfalls anschließend an die so erhaltenen Verbindungen der Formel (I) eine Säure oder ein Metallsalz addiert.

3. Pflanzenwuchsregulierende und fungizide Mittel, gekennzeichnet durch einen Gehalt an mindestens einem substituierten Azolylcyclopropyl-azolylmethyl-carbinol-Derivat oder Formel (I) bzw. einem Säureadditions-Salz oder Metallsalz-Komplex eines substituierten Azolylcyclopropyl-azolylmethyl-carbinol-Derivates der Formel (I).

4. Verwendung von substituierten Azolylcyclopropyl-azolylmethyl-carbinol-Derivaten der Formel (I) bzw. von deren Säureadditions-Salzen und Metallsalz-Komplexen zur Regulierung des Pflanzenwachstums un zur Bekämpfung von Pilzen.

5. Aryl-azolylcyclopropyl-ketone der Formel

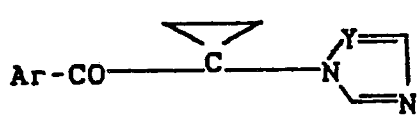

(II)

in welcher
a) Y für eine CH-Gruppe steht und
Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy,

gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

b) Y für ein Stickstoffatom steht und

Ar für Phenyl steht, das einfach oder zweifach, gleichartig oder verschieden substituiert sein kann durch Methylthio, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht.

6. Verfahren zur Herstellung von Aryl-azolylcyclopropyl-ketonen der Formel

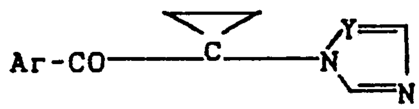

(II)

in welcher

a) Y für eine CH-Gruppe steht und

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

b) Y für ein Stickstoffatom steht und

Ar für Phenyl steht, das einfach oder zweifach, gleichartig oder verschieden substituiert sein kann durch Methylthio, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, dadurch gekennzeichnet, daß man Aryl-halogenpropylketone der Formel

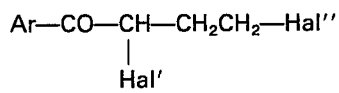

(VII)

in welcher

Ar die oben angegebene Bedeutung hat und Hal' und Hal'' für Halogen stehen, mit Azolen der Formel

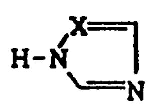

(V)

in welcher

Y die oben angegebene Bedeutung hat, in Gegenwart eines Verdünnungsmittels und in Gegenwart einer Base umsetzt.

7. Aryl-azolylcyclopropyl-oxirane der Formel

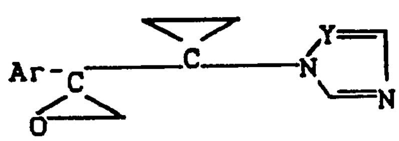

(IV)

in welcher

a) Y für eine CH-Gruppe steht und

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

b) Y für ein Stickstoffatom steht und

Ar für Phenyl steht, das einfach oder zweifach, gleichartig oder verschieden substituiert sein kann durch Methylthio, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht.

8. Verfahren zur Herstellung von Aryl-azolylcyclopropyl-oxiranen dr Formel

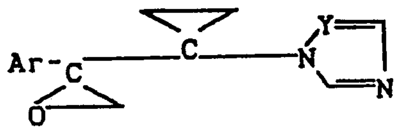

(IV)

in welcher

a) Y für eine CH-Gruppe steht und

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

b) Y für ein Stickstoffatom steht und

Ar für Phenyl steht, das einfach oder zweifach, gleichartig oder verschieden substituiert sein kann durch Methylthio, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, dadurch gekennzeichnet, daß man Aryl-azolylcyclopropyl-ketone der Formel

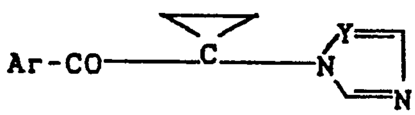

(II)

in welcher

Ar und Y die oben angegebene Bedeutung haben, mit Dimethyloxosulfonium-methylid der Formel

$$\overset{\delta+\ \delta-}{(CH_3)_2SOCH_2} \qquad (III)$$

in Gegenwart eines Verdünnungsmittels umsetzt.

9. Di-azolyl-keto-Derivate der Formel

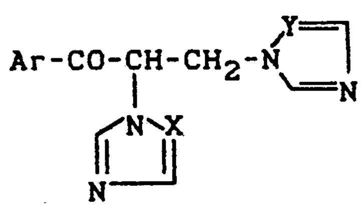

(VI)

in welcher

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy, gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht, oder

X für Stickstoff oder eine CH-Gruppe steht und

Y für Stickstoff oder eine CH-Gruppe steht.

10. Verfahren zur Herstellung von Di-azolyl-keto-Derivaten der Formel

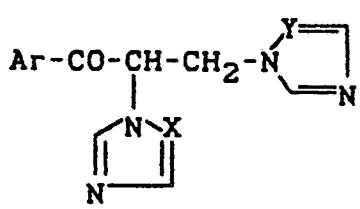

(VI)

in welcher

Ar für Phenyl steht, das einfach oder zweifach, gleichartig oder verschieden substituiert sein kann durch Fluor, Chlor, Methyl, Isopropyl, tert.-Butyl, Methoxy, Methylthio, Trifluormethyl, Trifluormethoxy,

gegebenenfalls durch Fluor, Chlor und/oder Methyl substituiertes Phenyl oder gegebenenfalls durch Fluor, Chlor und/oder Methyl substituiertes Phenoxy, oder

Ar für Naphthyl steht,

X für Stickstoff oder eine CH-Gruppe steht und

Y für Stickstoff oder eine CH-Gruppe steht, dadurch gekennzeichnet, daß man Aryl-azolylmethylketone der Formel

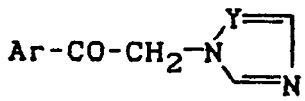

(IX)

in welcher

Ar und Y die oben angegebene Bedeutung haben, mit Hydroxymethylazolen der Formel

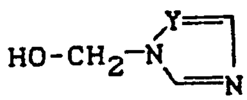

(X)

in welcher

X die oben angegebene Bedeutung hat, in Gegenwart eines Katalysators und in Gegenwart eines Verdünnungsmittels umsetzt.

**Revendications**

1. Azolylcyclopropyl-azolylméthyl-carbinols substitués de formule:

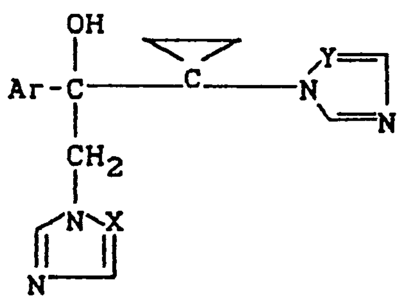

(I)

dans laquelle

a) X représente un atome d'azote ou un groupe CH,

Y représente un groupe CH et

Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore, et/ou des groupes méthyle, ou bien

Ar représente un groupe naphtyle, ou bien

b) X représente un groupe CH,

Y représente un atome d'azote et

Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle, ou bien

c) X représente un atome d'azote,

Y représente un atome d'azote et

Ar représente un groupe phényle qui peut porter un ou deux substituants identiques ou différents choisis parmi les groupes méthylthio, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle, ainsi que leurs sels formés par addition avec des acides et complexes de sels métalliques.

2. Procédé de préparation des azolylcyclopropyl-azolylméthylcarbinols substitués de formule:

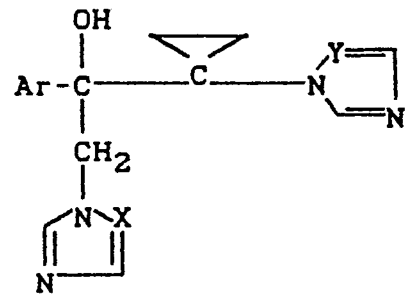

(I)

dans laquelle

a) X représente un atome d'azote ou un groupe CH,
Y représente un groupe CH et
Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien
Ar représente un groupe naphtyle, ou bien

b) X représente un groupe CH,
Y représente un atome d'azote et
Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien
Ar représente un groupe naphtyle, ou bien

c) X représente un atome d'azote,
Y représente un atome d'azote et
Ar représente un groupe phényle qui peut porter un ou deux substituants identiques ou différents choisis parmi les groupes méthylthio, trifluorométhoxy, phényle éventuellement substitué par le fluor, le chlore et/ou des groupes méthyle, ou phénoxy éventuellement substitué par le fluor, le chlore et/ou des groupes méthyle, ou bien
Ar représente un groupe naphtyle, ainsi que des sels formés par addition avec des acides et complexes de sels métalliques des composés de formule I, caractérisé en ce que

a) dans un premier stade opératoire, on fait réagir des aryl-azolylcyclopropyl-cétones de formule:

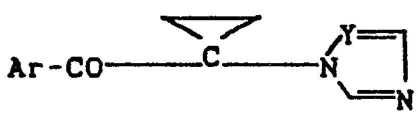

(II)

dans laquelle
Ar et Y ont les significations indiquées ci-dessus, avec le méthylure de diméthyloxosulfonium de formule:

$$\overset{\delta+\ \delta-}{(CH_3)_2SOCH_2} \qquad (III)$$

en présence d'un diluant, ce qui donne des aryl-azolylcyclopropyloxirannes de formule:

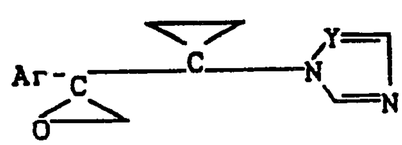

(IV)

dans laquelle

Ar et Y ont les significations indiquées ci-dessus, qu'on fait réagir dans un deuxième stade opératoire avec des azoles de formule:

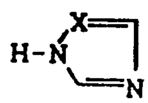

(V)

dans laquelle

X a les significations indiquées ci-dessus, en présence d'un diluant et en présence d'une base; ou bien

b) on fait réagir des dérivés diazolyl-cétoniques de formule:

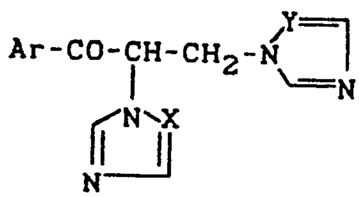

(VI)

dans laquelle

Ar, X et Y ont les significations indiquées ci-dessus, avec le méthylure de diméthyloxosulfonium de formule:

$$\overset{\delta+}{(CH_3)_2SO}\overset{\delta-}{CH_2} \quad \text{(III)}$$

en présence d'une diluant, après quoi, le cas échéant, on fixe sur les composés ainsi obtenus, répondant à la formule I, par addition, un acide ou und sel métallique.

3. Produits fongicides et régulateurs de la croissance des végétaux, caractérisés en ce qu'ils contiennent au moins un azolylcyclopropyl-azolylméthyle-carbinol substitué de formule I ou un sel formé par addition avec un acide ou un complexe de sel métallique d'un azolylcyclopropyl-azolylméthyl-carbinol substitué de formule I.

4. Utilisation des azolylcyclopropyl-azolylméthylcarbinols substitués de formule I ou de leurs sels formés par addition avec des acides et complexes de sels métalliques pour la régulation de la croissance des végétaux et la lutte contre les mycètes.

5. Aryl-azolylcyclopropyl-cétones de formule:

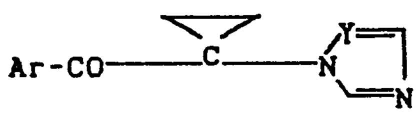

(II)

dans laquelle

a) Y représente un groupe CH et

Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle, ou bien

b) Y représente un atome d'azote et

Ar représente un groupe phényle qui peut porter un ou deux substituants identiques ou différents choisis parmi les groupes méthylthio, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle.

6. Procédé de préparation des aryl-azolylcyclopropyl-cétones de formule:

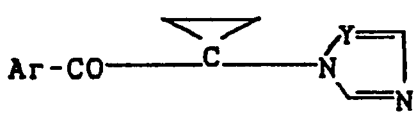

(II)

dans laquelle

a) Y représente un groupe CH et

Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle, ou bien

b) Y représente un atome d'azote et

Ar représente un groupe phényle qui peut porter un ou deux substituants identiques ou différents choisis parmi les groupes méthylthio, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle, caractérisé en ce que l'on fait réagir des aryl-halogénopropyl-cétones de formule:

$$Ar—CO—\underset{\displaystyle Hal'}{\underset{|}{CH}}—CH_2CH_2—Hal'' \qquad (VII)$$

dans laquelle

Ar a les significations indiquées ci-dessus et

Hal' et Hal'' représentent des halogènes, avec des azoles de formule:

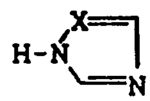

(V)

dans laquelle

Y a les significations indiquées ci-dessus, en présence d'un diluant et en présence d'une base.

7. Aryl-azolylcyclopropyl-oxirannes de formule:

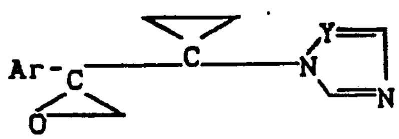

(IV)

dans laquelle

a) Y représente un groupe CH et

Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle, ou bien

b) Y représente un atome d'azote et

Ar représente un groupe phényle qui peut porter un ou deux substituants identiques ou différents choisis parmi les groupes méthylthio, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle.

8. Procédé de préparation des aryl-azolylcyclopropyl-oxirannes de formule:

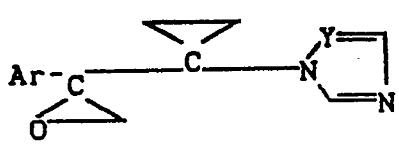

(IV)

dans laquelle

a) Y représente un groupe CH et

Ar représente un groupe phényle qui peut porter un ou deux substituants identiques ou différents

choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle, ou bien

b) Y représente un atome d'azote et

Ar représente un groupe phényle qui peut porter un ou deux substituants identiques ou différents choisis parmi les groupes méthylthio, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle, caractérisé en ce que l'on fait réagir es aryl-azolylcyclopropyl-cétones de formule:

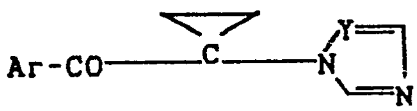

(II)

dans laquelle

Ar et Y on les significations indiquées ci-dessus, avec le méthylure de diméthyloxosulfonium de formule:

$$\overset{\delta+\ \delta-}{(CH_3)_2SOCH_2} \qquad (III)$$

en présence d'un diluant.

9. Dérivé diazolyl-cétoniques de formule:

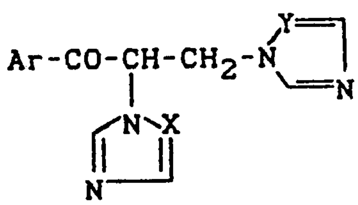

(VI)

dans laquelle

Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle,

X représente l'azote ou un groupe CH et

Y représente l'azote ou un groupe CH.

10. Procédé de préparation des dérivés diazolylcétoniques de formule:

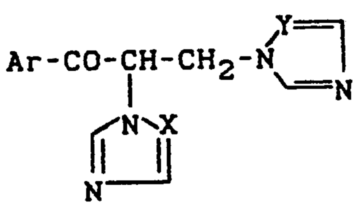

(VI)

dans laquelle

Ar représente un groupe phényle qui peut porter un ou deux substituants identiques ou différents choisis parmi le fluor, le chlore, les groupes méthyle, isopropyle, tert-butyle, méthoxy, méthylthio, trifluorométhyle, trifluorométhoxy, phényle éventuellement substitué par le fluor, le chlore et/ou des groupes méthyle, ou phénoxy éventuellement substitué par le fluor, le chlore et/ou des groupes méthyle, ou bien

Ar représente un groupe naphtyle,

X représente l'azote ou un groupe CH et

Y représente l'azote ou un groupe CH, caractérisé en ce que l'on fait réagir des aryl-azolylméthylcétones de formule:

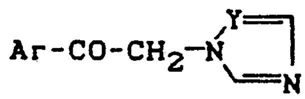

(IX)

dans laquelle

Ar et Y ont les significations indiquées ci-dessus, avec des hydroxyméthylazoles de formule:

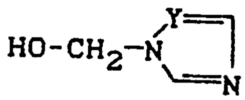

(X)

dans laquelle

X a les significations indiquées ci-dessus, en présence d'un catalyseur et en présence d'un diluant.

**Claims**

1. Substituted azolylcyclopropyl-azolylmethyl-carbinol derivaties of the formula

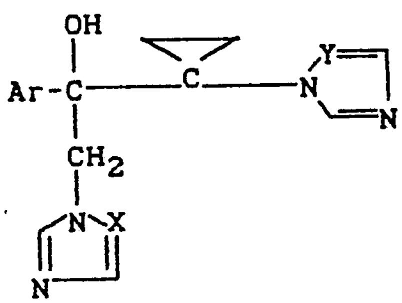

(I)

in which

a) X represents a nitrogen atom or a CH group,
Y represents a CH group and
Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or
Ar represents naphthyl, or

b) X represents a CH group,
Y represents a nitrogen atom and
Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or
Ar represents naphthyl, or

c) X represents a nitrogen atom,
Y represents a nitrogen atom and
Ar represents phenyl which can be monosubstituted or disubstituted by identical or different substituents from the series comprising methylthio, trifluoromethoxy, phenyl which is optionally substituted by fluorine, chlorine and/or methyl, or phenoxy which is optionally substituted by fluorine, chlorine and/or methyl, or
Ar represents nahthyl, and their acid addition salts and metal salt complexes.

2. Process for the preparation of substituted azolyl-cyclopropyl-azolylmethyl-carbinol derivatives of the formula

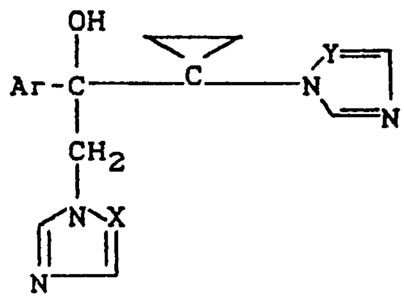

(I)

in which

a) X represents a nitrogen atom or a CH group,
Y represents a CH group and
Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or
Ar represents naphthyl, or

b) X represents a CH group,
Y represents a nitrogen atom and
Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or
Ar represents naphthyl, or

c) X represents a nitrogen atom,
Y represents a nitrogen atom and
Ar represents phenyl which can be monosubstituted or disubstituted by identical or different substituents from the series comprising methylthio, trifluoromethoxy, phenyl which is optionally substituted by fluorine, chlorine and/or methyl, or phenoxy which is optionally substituted by fluorine, chlorine and/or methyl, or
Ar represents naphthyl, and of acid addition salts and metal salt complexes of compounds of the formula (I), characterized in that

a) in a first stage, aryl azolylcyclopropyl ketones of the formula

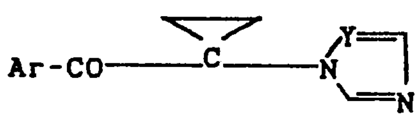

(II)

in which
Ar and Y have the meaning given above, are reacted with dimethyloxosulphonium methylide of the formula

$$\overset{\delta+\ \delta-}{(CH_3)_2SOCH_2} \qquad (III)$$

in the presence of a diluent, and, in a second stage, the resulting aryl-azolylcyclopropyl-oxiranes of the formula

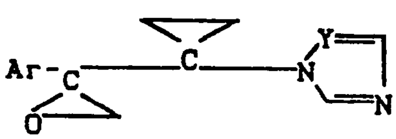

(IV)

in which
Ar and Y have the meaning given above, are reacted with azoles of the formula

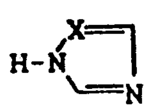

(V)

in which

X has the meaning given above, in the presence of a diluent and in the presence of a base; or

b) diazolyl-keto derivatives of the formula

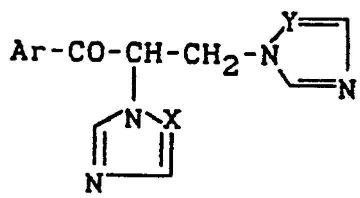

(VI)

in which

Ar, X and Y have the meaning given above, are reacted with dimethyloxosulphonium methylide of the formula

$$\overset{\delta+\ \delta-}{(CH_3)_2SOCH_2} \qquad (III)$$

in the presence of a diluent and, if appropriate, the resulting compounds of the formula (I) are subjected to an addition reaction with an acid or a metal salt.

3. Plant growth-regulating and fungicidal agents, characterized in that they contain at least one substituted azolylcyclopropyl-azolylmethyl-carbinol derivative of the formula (I) or one acid addition salt or metal salt complex of a substituted azolylcyclopropyl-azolylmethyl-carbinol derivative of the formula (I).

4. Use of substituted azolylcyclopropyl-azolyl-methyl-carbinol derivatives of the formula (I) or of their acid addition salts and metal salt complexes for regulating plant growth and for combating fungi.

5. Aryl azolylcyclopropyl ketones of the formula

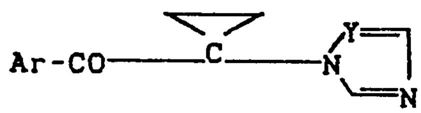

(II)

in which

a) Y represents a CH group and

Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or

Ar represents naphthyl, or

b) Y represents a nitrogen atom and

Ar represents phenyl which can be monosubstituted or disubstituted by identical or different substituents from the series comprising methylthio, trifluoromethoxy, phenyl which is optionally substituted by fluorine, chlorine and/or methyl, or phenoxy which is optionally substituted by fluorine, chlorine and/or methyl, or

Ar represents naphthyl.

6. Process for the preparation of aryl azolylcyclopropyl ketones of the formula

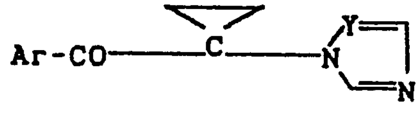

(II)

in which

a) Y represents a CH group and

Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or

Ar represents naphthyl, or

b) Y represents a nitrogen atom and

Ar represents phenyl which can be monosubstituted or disubstituted by identical or different substituents from the series comprising methylthio, trifluoromethoxy, phenyl which is optionally

substituted by fluorine, chlorine and/or methyl, or phenoxy which is optionally substituted by fluorine, chlorine and/or methyl, or

Ar represents naphthyl, characterized in that aryl halogenopropyl ketones of the formula

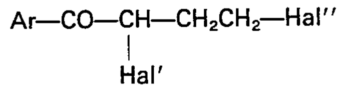

(VII)

in which

Ar has the meaning given above the Hal' and Hal'' represent halogen, are reacted with azoles of the formula

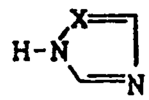

(V)

in which

Y has the meaning given above, in the presence of a diluent and in the presence of a base.

7. Aryl-azolylcyclopropyl-oxiranes of the formula

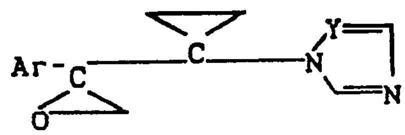

(IV)

in which

a) Y represents a CH group and

Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or

Ar represents naphthyl, or

b) Y represents a nitrogen atom and

Ar represents phenyl which can be monosubstituted or disubstituted by identical or different substituents from the series comprising methylthio, trifluoromethoxy, phenyl which is optionally substituted by fluorine, chlorine and/or methyl, or phenoxy which is optionally substituted by fluorine, chlorine and/or methyl, or

Ar represents naphthyl.

8. Process for the preparation of aryl-azolylcyclopropyl-oxiranes of the formula

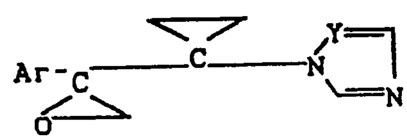

(IV)

in which

a) Y represents a CH group and

Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or

Ar represents naphthyl, or

b) Y represents a nitrogen atom and

Ar represents phenyl which can be monosubstituted or disubstituted by identical or different substituents from the series comprising methylthio, trifluoromethoxy, phenyl which is optionally substituted by fluorine, chlorine and/or methyl, or phenoxy which is optionally substituted by fluorine, chlorine and/or methyl, or

Ar represents naphthyl, characterized in that aryl azolylcyclopropyl ketones of the formula

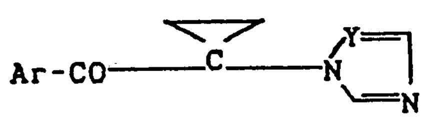

(II)

in which

Ar and Y have the meaning given above, are reacted with dimethyloxosulphonium methylide of the formula

$$\overset{\delta+\ \delta-}{(CH_3)_2SOCH_2} \qquad (III)$$

in the presence of a diluent.

9. Di-azolyl-keto derivatives of the formula

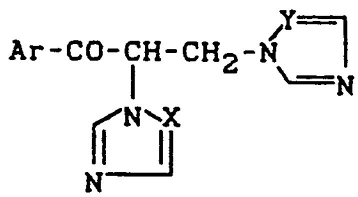

(VI)

in which

Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or

Ar represents naphthyl,

X represents nitrogen or a CH group and

Y represents nitrogen or a CH group.

10. Process for the preparation of di-azolyl-keto derivatives of the formula

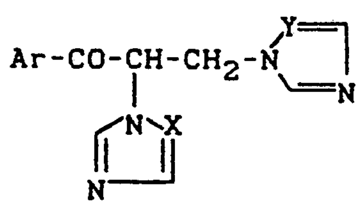

(VI)

in which

Ar represents phenyl which can be monosubstituted or disubstituted by identical or different substituents from the series comprising fluorine, chlorine, methyl, isopropyl, tert.-butyl, methoxy, methylthio, trifluoromethyl, trifluoromethoxy, phenyl which is optionally substituted by fluorine, chlorine and/or methyl, or phenoxy which is optionally substituted by fluorine, chlorine and/or methyl, or

Ar represents naphthyl,

X represents nitrogen or a CH group and

Y represents nitrogen or a CH group, characterized in that aryl azolylmethyl ketones of the formula

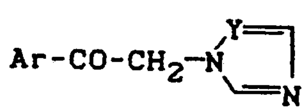

(IX)

in which

Ar and Y have the meaning given above, are reacted with hydroxymethylazoles of the formula

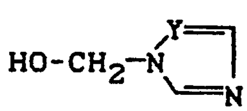

(X)

in which

X has the meaning given above, in the presence of a catalyst and in the presence of a diluent.